# EUROPÄISCHE PATENTSCHRIFT

(11) **EP 0 352 252 B1**
(45) Veröffentlichungstag und Bekanntmachung des Hinweises auf die Patenterteilung: **03.11.1993**
(21) Anmeldenummer: 89890192.1
(22) Anmeldetag: 18.07.1989
(51) Int. Cl.: F16H 13/08, A61F 2/68

(54) **Planetenreibrädergetriebe**
Epicyclic friction gear
Engrenage planétaire à friction

(30) Priorität: 18.07.1988 AT 1843/88
(43) Veröffentlichungstag der Anmeldung: 24.01.1990
(73) Patentinhaber: Otto Bock Orthopädische Industrie Besitz- und Verwaltungs-Kommanditgesellschaft, 37115 Duderstadt (DE)
(72) Erfinder: Horvath, Eduard, A-1070 Wien (AT)
(74) Vertreter: Casati, Wilhelm, Dipl.-Ing.

(56) Entgegenhaltungen:
- DE-A- 2 121 023
- DE-C- 892 536
- GB-A- 354 761
- GB-A- 413 802
- GB-A- 2 002 066
- US-A- 1 737 295

## Beschreibung

Die Erfindung bezieht sich auf ein Planetenreibrädergetriebe, gemäß dem Oberbegriff des Ansprüches 1.

Bei einer bekannten Ausbildung dieser Art (GB-A-354 761) sind Planetenräder mit konischen Mantelflächen vorgesehen, wobei die Planetenräder an der Mitnehmerplatte über Achsen starr festgehalten sind. Die Mitnehmerplatte ist dabei im Bereich der Halterung für die Planetenräder etwas federnd ausgebildet. Bei derartigen Ausbildungen ist es unumgänglich notwendig, daß sich die Erzeugenden der Mantelflächen aller Planetenräder sowie auch die Achsen aller Planetenräder in einem gemeinsamen Punkt auf der Längsachse der Antriebswelle schneiden, da nur dann ein gleichmäßiger Anpreßdruck der Planetenräder am Sonnenrad vorhanden ist. Außerdem muß die Konusfläche des Hohlrades hinsichtlich der Winkellage ebenfalls so angeordnet sein, daß einerseits die Erzeugenden der Konusfläche des Hohlrades mit den Erzeugenden der konischen Mantelflächen der Planetenräder zusammenfallen, und andererseits der Abstand zwischen dem Sonnenrad und dem konischen Bereich des Hohlrades exakt dem Durchmesser der Planetenräder entspricht. Bei dieser bekannten Ausbildung ist zu bedenken, daß sich aufgrund der federnden Ausbildung der Mitnehmerplatte die Winkellage der Tragachsen der Planetenräder verschwenken kann, so daß dann ein Zusammenfallen der Erzeugenden der konischen Mantelflächen der Planetenräder mit den Erzeugenden des Sonnenrades bzw. des konischen Bereiches des Hohlrades nicht mehr gegeben ist, so daß eine "punktförmige" Anlage des Planetenrades an der Abrollfläche gegeben ist. Weiters ist bei der bekannten Ausbildung zu bedenken, daß derartige Ausbildungen nicht beliebig verkleinerbar sind, da die Fertigungstoleranzen einen gewissen Wert nicht unterschreiten können, so daß das Spiel bzw. Klemmkräfte aufgrund der Verkleinerung zunehmen können.

Der Erfindung liegt die Aufgabe zugrunde, ein Planetenreibrädergetriebe der eingangs genannten Art so auszubilden, daß der Anpreßdruck der Planetenräder an der Antriebswelle präzise einstellbar ist, wobei Fertigungstoleranzen aufgrund der Konstruktion ausgeglichen werden können.

Erfindungsgemaß wird diese Aufgabe dürch die Merkmale des Kennzeichnenden Teils des Ansprüches 1 gelöst,. Dadurch wird erreicht, daß der Anpreßdruck, mit welchem die Planetenräder an den Abrollflächen gehalten werden, direkt mittels des Hohlrades erreicht wird, wobei aufgrund der Einstellbarkeit des Hohlrades eine gleichmäßige Verteilung auf alle Planetenräder erfolgt. Bei der erfindungsgemäßen Ausbildung werden nämlich die Planetenräder nicht durch eine spezielle starre Achse geführt, sondern laufen aufgrund ihrer zylindrischen Ausbildung gleichmäßig am Außenmantel des Sonnenrades um, wobei sie sich selbst so ausrichten, daß die Erzeugenden des Außenmantels der Planetenräder mit den Erzeugenden des Sonnenrades zusammenfallen. Weiters sind die Erzeugenden aller Abrollflächen der Planetenräder zueinander parallel. Die Mitnehmerstifte greifen nur lose in die zentralen Bohrungen der Planetenräder ein und beeinflussen damit die Ausrichtung der Planetenräder in keiner Weise. Auf diese Weise ist es möglich, Planetenreibrädergetriebe mit einem Gesamtdurchmesser von 10 mm zu bauen, also sehr stark zu miniaturisieren, so daß des erfindungsgemäße Planetenreibrädergetriebe in Prothesenantriebe einbaubar ist, wo es bekanntlich in räumlicher Beziehung sehr beschränkt ist.

Es ist an sich bekannt, siche (DE-A-2121023) daß die Planetenräder zylindrischen Außenmantel aufweisen. Bei dieser bekannten Ausbildung ist jedoch die Abrollfläche des Sonnenrades und auch die Abrollfläche des Hohlrades zylindrisch ausgebildet, so daß die Wahl bzw. Einstellung des Anpreßdruckes der Planetenräder insofern sehr schwierig ist, als ein einmal vorgegebener Wert nicht mehr geändert werden kann. Bei zu geringem Anpreßdruck kommt es zu einem Durchrutschen der Antriebswelle, bei zu starkem Anpreßdruck hingegen zu Materialschädigungen bzw. Materialverformungen. Aufgrund von Fertigungstoleranzen kann ein Einlaufen der Antriebswelle auftreten, d.h. daß sich in der Antriebswelle umlaufende Rillen bilden können, die auf eine Verkantung der Planetenräder zurückgehen, wobei dann die Antriebswelle auf Zug oder auch auf Schub beansprucht werden kann.

Vorteilhafterweise ist nach der Erfindung das Hohlrad an einer konzentrisch zur Antriebswelle feststehend angeordneten Hülse angebracht, welche auf ein zugehöriges Tragorgan aufschraub- und in der gewünschten Lage festlegbar ist. Dadurch kann eine direkte Einstellung des Hohlrades in bezug auf das Sonnenrad vorgenommen werden, ohne daß die Antriebswelle verstellt werden muß. Bei der Ausbildung nach der Gattung hingegen muß zur Einstellbarkeit des Abstandes zwischen dem Sonnenrad und dem Hohlrad die gesamte Lagerung der Antriebswelle im Gehäuse axial verschoben werden.

Weiters kann die dem Sonnenrad gegenüberliegende Abrollflache des Hohlrades, an der die Planetenräder abrollen, ballig ausgebildet sein, wodurch erreicht wird, daß sich die Planetenräder noch besser in bezug auf die Anlage am Sonnenrad einstellen können. Um ein unerwünschtes Abgleiten der Planetenräder vom Hohlrad zu verhindern, kann die Abrollfläche für die Planetenräder am Hohlrad, vorzugsweise beidseits, durch radial nach innen gerichtete Ringflansche begrenzt sein.

Bei einer weiteren Ausführungsvariante kann der das Sonnenrad bildende konische Bereich der Antriebswelle gegenüber einer Motorwelle axial verschiebbar, jedoch mit dieser drehschlüssig verbunden sein, wobei in an sich bekannter Weise der das Sonnenrad bildende konische Bereich bezüglich des Hohlrades über ein axiale Kräfte aufnehmendes Stützlager am Tragorgan für die das Hohlrad tragende Hülse abgestützt ist. Durch die verschiebbare, jedoch drehschlüssige Verbindung zwischen Antriebswelle und Motorwelle wird erreicht, daß die Motorwelle von axial wirkenden Kräften freigehalten wird, die durch die Planetenräder auf den das Sonnenrad bildende konischen Bereich übertragen werden können. Die auftretenden Axialkräfte werden dabei durch das die axialen Kräfte aufnehmende Stützlager direkt in das Tragorgan abgeleitet. Um einen vorgegebenen, gleichbleibenden Anpreßdruck zu erzielen, kann das Stützlager einen, vorzugsweise durchgehenden, radial nach außen abstehenden Flansch aufweisen, an welchem eine Druckfeder anliegt, die sich mit ihrem anderen Ende am Tragorgan abstützt. Durch entsprechendes Verstellen des Hohlrades wird damit die Feder in gewünschtem Maß vorgespannt bzw. entlastet, wodurch für die Anpreßkraft lediglich die Federkraft maßgebend ist, so daß thermische Faktoren die Anpreßkraft nicht beeinflussen können.

In der Zeichnung sind zwei Ausführungsbeispiele im Axialschnitt dargestellt, wobei gemäß Fig. 1 der die das Sonnenrad bildende konische Anlageflache tragende Teil der Antriebswelle über ein Stützlager direkt am Tragorgan für die Hülse des Sonnenrades abgestützt ist. Beim Ausführungsbeispiel gemäß Fig. 2 ist das Stützlager über eine Feder am Tragorgan abgestützt. Fig. 3 zeigt eine Frontansicht des Planetengetriebes.

Mit 1 ist der Bereich der Antriebswelle bezeichnet, die zur Anlage der Planetenräder 3 bestimmt ist, wobei die Anlagefläche 2 für die Planetenräder konisch ausgebildet ist. Dieser konische Bereich bildet das Sonnenrad des Getriebes. Die Planetenräder 3 werden über ein Hohlrad 5 in Anlage an der konischen Anlagefläche 2 des das Sonnenrad bildenden Bereiches 1 der Antriebswelle gehalten, wobei die Anlagefläche 8 des Hohlrades 5 ballig nach innen gewölbt ist. Es könnte, in nicht dargestellter Weise, die Abrollfläche am Hohlrad auch als Kegelstumpfmantelfläche ausgebildet sein. Das Hohlrad 5 ist mit einer Hülse 6 verbunden, welche auf ein Tragorgan 7 aufschraubbar ist, das in bezug auf den Antriebsmotor 11 feststehend ausgebildet ist. Die vom Antriebsmotor 11 wegführende Motorwelle 13 ist mit dem Bereich 1 der Antriebswelle über eine Zahnkupplung 12 verbunden, welche eine Axialverschiebung zwischen dem Bereich 1 und der Motorwelle 13 zuläßt, jedoch eine drehschlüssige Verbindung zwischen den beiden Teilen ergibt. Der Bereich 1 der Antriebswelle ist in einem, die Verzahnung der Kupplung 12 tragenden Teil 14 festgelegt, welcher sich über eine Schulter 15 an einem Stützlager 10 abstützt, welches mit seiner anderen Seite am Tragorgan 7 in Anlage ist. Die Planetenräder sind an der balligen Fläche 8 des Hohlrades 5 über radial nach innen vorspringende Ringflansche 9 gegen ein Abgleiten gesichert. Die Planetenräder 3 sind vorliegend als Kugellager ausgebildet, in deren zentrale Bohrung Mitnehmerstifte 16 eingreifen, die mit einer Mitnehmerplatte 17 in fester Verbindung stehen, über welche die Bewegung der Planetenräder abgenommen wird. Die Achse der Mitnehmerstifte 16 stimmt dabei mit der Achse 4 der Planetenräder 3 nicht überein, was jedoch insofern unmaßgeblich ist, als durch entsprechende Wahl der Dicke der Mitnehmerstifte 16 verhindert wird, daß durch letztere Kippmomente auf die Planetenräder 3 übertragen werden.

Zur Einstellung des Anpreßdruckes der Planetenräder 3 an der konischen Außenfläche 2 des das Sonnenrad bildenden Bereiches 1 der Antriebswelle wird die Hülse 6 auf das Tragorgan 7 entsprechend weit aufgeschraubt, wodurch aufgrund der Pressung zwischen der balligen Anlagefläche 8 und der konischen Anlagefläche 2 die Planetenräder 3 mit entsprechendem Druck in Anlage gebracht werden, um so eine entsprechende Mitnahmekraft zu erzielen. Der Gegendruck zwischen der konischen Anlagefläche 2 am das Sonnenrad bildenden Bereich 1 der Antriebswelle wird über das Stützlager 10 und das Tragorgan 7 erzeugt.

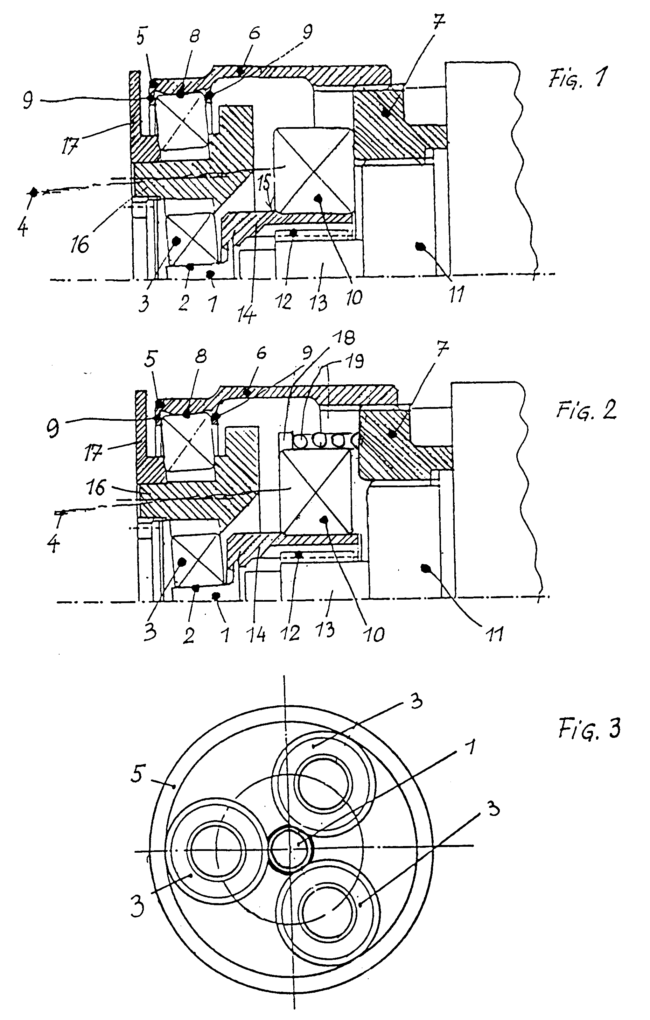

Beim Ausführungsbeispiel nach Fig. 2, welches grundsätzlich gleich dem Ausführungsbeispiel 1 aufgebaut ist, weist das Stützlager 10 seitliche, radial nach außen vorspringende Flansche 18 auf, an welchen sich eine Feder 19 abstützt, die mit ihrem anderen Ende an der Vorderfläche des Tragorgans 7 anliegt. Zwischen dem Tragorgan 7 und dem Stützlager 10 ist ein Zwischenraum freigelassen, so daß der Anpreßdruck des als Sonnenrad dienenden Bereiches 1 an die Planetenräder 3 lediglich durch die Kraft der Feder 19 erzeugt wird. Aufgrund der Vorwahl der Spannung der Feder 19 kann damit ein immer gleichbleibender Anpreßdruck hervorgerufen werden.

Mit Hilfe des erfindungsgemäßen Kegelreib-Getriebes wird erreicht, daß insbesondere beim Antrieb von Prothesen oder sonstigen künstlichen Gelenken ein auf kleinem Raum gegebenes Reduktionsgetriebe vorliegt, von welchem die Kraft bzw. Bewegung in axialer Richtung abgenommen werden kann.

Die Achsen 4 der Planetenräder sind gemäß dem Ausführungsbeispiel parallel zu den Erzeugenden der konischen Anlagefläche 2 für die Planetenräder 3 angeordnet. Im allgemeinen sind die Achsen der Planetenräder 3 zur Achse der Antriebswelle geneigt. Bei Parallelität mit der Achse der Antriebswelle würde eine hohe Kantenpressung auftreten.

## Patentansprüche

1. Planetenreibrädergetriebe, insbesondere miniaturisiertes Getriebe für Prothesenantriebe, bei welchem Planetenräder (3) an einem als Sonnenrad dienenden konischen Bereich einer Antriebswelle (1) und einem in bezug auf die Antriebswelle stillstehenden Hohlrad abrollen, bei dem eine mit den Planetenrädern über Mitnehmerstifte (16) in Verbindung stehende Mitnehmerplatte (17) an einer Abtriebswelle angeordnet ist, und bei dem das Hohlrad und das Sonnenrad in Achsrichtung zueinander verstell- und gegeneinander festlegbar sind, dadurch gekennzeichnet, daß die Planetenräder (3) in an sich bekannter Weise zylindrische Außenmäntel aufweisen, daß die auf der Mitnehmerplatte angeordneten Mitnehmerstifte (16) lose in zentrale Bohrungen der Planetenräder (3) eingreifen, und daß die Planetenräder (3) mittels des Hohlrades (5) an dem Sonnenrad über die gesamte Länge der Erzeugenden der zylindrisehen Außenmantelfläche der Planetenräder (3) in Anlage gehalten sind.

2. Planetenreibrädergetriebe nach Anspruch 1, dadurch gekennzeichnet, daß das Hohlrad (5) an einer konzentrisch zur Antriebswelle feststehend angeordneten Hülse (6) angebracht ist, welche auf ein zugehöriges Tragorgan (7) aufgeschraubt und in der gewünschten Lage festlegbar ist.

3. Planetenreibrädergetriebe nach einem der Ansprüche 1 oder 2, dadurch gekennzeichnet, daß die dem Sonnenrad gegenüberliegende Abrollfläche (8) des Hohlrades (5), an der die Planetenträder (3) abrollen, ballig ausgebildet ist.

4. Planetenreibrädergetriebe nach Anspruch 3, dadurch gekennzeichnet, daß die Abrollfläche (8) für die Planetenräder (3) am Hohlrad (5), vorzugsweise beidseits, durch radial nach innen gerichtete Ringflansche (9) begrenzt ist.

5. Planetenreibrädergetriebe nach einem der Ansprüche 1 bis 4, dadurch gekennzeichnet, daß der das Sonnenrad bildende konische Bereich (1) der Antriebswelle (1) gegenüber einer Motorwelle (13) axial verschiebbar, jedoch mit dieser drehschlüssig verbunden ist, wobei in an sich bekannter Weise der als Sonnenrad ausgebildete konische Bereich (1) bezüglich des Hohlrades (5) über ein axiale Kräfte aufnehmendes Stützlager (10) am Tragorgan (7) für die das Hohlrad (S) tragende Hülse (6) abgestützt ist.

6. Planetenreibrädergetriebe nach Anspruch 5, dadurch gekennzeichnet, daß das Stützlager (10) einen, vorzugsweise durchgehenden, radial nach außen abstehenden Flansch (18) aufweist, an welchem eine Druckfeder (19) anliegt, die sich mit ihrem anderen Ende am Tragorgan (7) abstützt.

## Claims

1. Planetary friction wheel mechanism, in particular a miniaturized mechanism for prosthesis drives, in which planet wheels (3) roll on a tapered area, serving as a sun wheel, of an input shaft (1) and on a ring wheel stationary in relation to the input shaft, in which a driver plate (17) connected to the planet wheels via driver pins (16) is arranged on an output shaft, and in which the ring wheel and the sun wheel can be adjusted and fixed relative to one another in the axial direction, characterized in that the planet wheels (3) have, in a manner known per se, cylindrical outer circumferential surfaces, in that the driver pins (16) arranged on the driver plate engage loosely in central holes in the planet wheels (3), and in that the planet wheels (3) are held in contact with the sun wheel over the entire length of the generator of the cylindrical outer circumferential surface of the planet wheels (3) by means of the ring wheel.

2. Planetary friction wheel mechanism according to Claim 1, characterized in that the ring wheel (5) is attached to a sleeve (6) which is arranged in a fixed manner concentrically to the input shaft, is screwed onto an associated supporting member (7) and can be fixed in the desired position.

3. Planetary friction wheel mechanism according to either of Claims 1 or 2, characterized in that the rolling-contact surface (8) of the ring wheel (5) opposite the sun wheel, against which surface the planet wheels (3) roll, is of convex design.

4. Planetary friction wheel mechanism according to Claim 3, characterized in that the rolling-contact surface (8) for the planet wheels (3) on the ring wheel (5) is bounded, preferably on both sides, by annular flanges (9) pointed radially inwards.

5. Planetary friction wheel mechanism according to one of Claims 1 to 4, characterized in that the tapered area (1) of the input shaft (1), the area forming the sun wheel, is axially displaceable relative to a motor shaft (13) but connected rotationally to the latter, the tapered area (1), the area designed as a sun wheel, being supported in a manner known per se in relation to the ring wheel (5) via a support bearing (10) on the supporting member (7) for the sleeve (6) supporting the ring wheel (5), the said bearing taking up axial forces.

6. Planetary friction wheel mechanism according to Claim 5, characterized in that the support bearing (10) has a, preferably continuous, flange (18) projecting radially outwards, against which there rests a compression spring (19) which is supported at its other end against the supporting member (7).

## Revendications

1. Engrenage planétaire à friction, en particulier engrenage miniaturisé pour l'actionnement de prothèses, dans lequel des satellites (3) roulent sur une zone conique d'un arbre menant (1) servant de planétaire et sur une couronne immobile par rapport à l'arbre menant, dans lequel une plaque d'entraînement (17), reliée aux satellites par l'intermédiaire de doigts d'entraînement (16), est disposée sur un arbre mené, et dans lequel la position relative de la couronne et du planétaire peut être modifiée et fixée en direction axiale, caractérisé en ce que les satellites (3) présentent, de façon connue en soi, des enveloppes extérieures cylindriques, en ce que les doigts d'entraînement (16) disposés sur la plaque d'entraînement s'engagent sans serrage dans des perçages centraux des satellites (3) et en ce que les satellites (3), au moyen de la couronne (5) sont maintenus en appui sur le planétaire, sur toute la longueur de la génératrice de la surface de l'enveloppe extérieure cylindrique des satellites (3).

2. Engrenage planétaire à friction selon la revendication 1, caractérisé en ce que la couronne (5) est disposée sur un manchon (6) monté fixement de façon concentrique à l'arbre menant, qui est vissé sur un organe porteur (7) associé et peut être fixé dans la position souhaitée.

3. Engrenage planétaire à friction selon l'une des revendications 1 ou 2, caractérisé en ce que la surface de roulement (8) de la couronne (5) qui est en regard du planétaire, sur laquelle les satellites roulent, est conformée en sphère.

4. Engrenage planétaire à friction selon la revendication 3, caractérisé en ce que la surface de roulement (8) de la couronne (5), destinée aux satellites (3), est limitée, de préférence des deux côtés, par des brides annulaires (9) orientées radialement vers l'intérieur.

5. Engrenage planétaire à friction selon l'une des revendications 1 à 4, caractérisé en ce que la zone (1) conique, formant le planétaire, de l'arbre menant (1) peut être déplacée axialement vis-à-vis d'un arbre de moteur (13), mais est liée en rotation avec celui-ci, la zone (1) conique formant le planétaire étant appuyée, de façon connue en soi, vis-à-vis de la couronne (5), par l'intermédiaire d'un palier de support (10) absorbant les forces axiales, sur l'organe porteur (7) destiné au manchon (6) portant la couronne (5).

6. Engrenage planétaire à friction selon la revendication 5, caractérisé en ce que le palier de support (10) présente une bride (18), de préférence continue, dépassant radialement vers l'extérieur, sur laquelle est monté un ressort de compression (19) qui s'appuie, par son autre extrémité, sur l'organe porteur (7).
